# EUROPEAN PATENT SPECIFICATION

(11) **EP 1 347 732 B1**
(45) Date of publication and mention of the grant of the patent: **14.06.2006**
(21) Application number: 02713344.6
(22) Date of filing: 03.01.2002
(51) Int. Cl.: A61K 6/06

(54) **DENTAL MATERIAL**
DENTALMATERIAL
MATERIAU DENTAIRE

(30) Priority: 04.01.2001 US 259685 P
(43) Date of publication of application: 01.10.2003
(73) Proprietor: DENTSPLY INTERNATIONAL, INC., York, PA 17405-0872 (US)
(72) Inventor: PRIMUS, Carolyn, M., Bradenton, FL 34203 (US)
(74) Representative: Hartz, Nikolai
(86) International application number: PCT/US2002/000016
(87) International publication number: WO 2002/056838

(56) References cited:
- US-A- 2 358 730
- US-A- 5 415 547
- DATABASE WPI Section Ch, Week 198412 Derwent Publications Ltd., London, GB; Class L02, AN 1984-072047 XP002199503 & JP 59 026961 A (FUJII M), 13 February 1984 (1984-02-13)

## Description

### TECHNICAL FIELD

The present invention is directed toward a dental material, such as a cement or a restorative material. More particularly, the invention relates to a dental material that is prepared with a Portland cement.

### BACKGROUND OF THE INVENTION

US Pat. No. 5,415,547 describes a composition of cement for dental applications. However, the composition of that Portland cement is gray in color. This color is deleterious in dental applications. The grayness of the cement produces a very un-esthetic result when the cement is visible through thin tissue, such as in the smaller teeth in pedodontics, or at the gum line. According to the present invention, white Portland cement can be substituted for such a gray Portland cement. White Portland cement has an advantage of being more similar in tooth color to teeth than the '547 patent. Therefore, the dark color from a conventional, gray Portland cement will not be present.

### SUMMARY OF THE INVENTION

It is an object of the invention to provide a dental material.

It is another object of the invention to provide a white dental material, such as a cement, a restorative or the like.

It is an additional object of the invention to provide such a dental material that contains Portland cement.

It is a still further object of the invention to provide such a material that is substantially free of iron oxide.

A white Portland cement according to the invention contains virtually no iron, unlike the '547 patent composition which contains about 5% iron oxide. Without iron oxide, the cement will have a white color, and fall within the compositional range of Portland cements, given as follows, all percents being by weight:
61 to 70% calcia
19 to 29 % silica,
5 to 15 % alumina and
0 to 0.5 % iron,
based upon 100 wt% of the material.

The material contains less than about 0.5 percent by weight of iron, based upon 100 percent by weight of the material.

### PREFERRED EMBODIMENTS FOR CARRYING OUT THE INVENTION

A dental material such as a cement, restorative or the like, according to the invention, has the following percentages by weight of components:
61 to 70% calcia
19 to 29 % silica,
5 to 15 % alumina and
0 to 0.5 % iron,
based on 100 wt % of the material.

The material contains less than about 0.5 percent by weight of iron, based upon 100 percent by weight of the material. More preferably, the material according to the invention is substantially free of iron oxide, meaning that it contains less than about 0.5 percent by weight of iron. Most preferably, the inventive material contains no iron. The material is visually observed to be white in color, and is therefore, more desirable in dental applications than gray-colored materials previously employed. A comparison of known gray-colored materials is provided in TABLE I.

| Table I | | |
|---|---|---|
| Normalized Composition of Cement Samples | | |
| Component | US '547 Patent | Colton Fast Set |
| Calcia | 65.00 | 64.2 |
| Silica | 21.00 | 20.8 |
| Iron oxide | 5.00 | 4.3 |
| Alumina | 4.00 | 3.9 |
| Magnesia | 2.00 | 3.2 |
| Sulfates | 2.50. | 2.6 |
| Soda, potassia | <0.5 | 0.6 |
| Titania | - | 0.2 |
| Phosphorous pentoxide | - | 0.09 |
| Manganese oxide | - | 0.05 |
| Strontia | - | 0.07 |
| LOI | - | 1.3 |
| As (ppm) | - | 16** |
| Pb (ppm) | - | 4.2** |

In TABLE I, Colton Fast Set cement is commercially available from the California Portland Cement Company. Analysis was conducted by x-ray fluorescence technique, normalized excluding LOI (loss on ignition at 950°C), and "ND" means "not detected."

Without iron oxide, the Portland cement has less of the calcium-alumino ferrite phase, as noted in Table II.

**Table II**

| Composition by Phase of Cement Samples | |
|---|---|
| Component | Exemplary Prior Material |
| 3CaO.SiO₂ | 62 |
| 2CaO.SiO₂ | 11 |
| 3CaO.Al₂O₃ | 3 |
| 4CaO.Al₂O₃.Fe₂O₃ | 13 |
| TOTAL Crystalline Phases Calculated from composition | 89 |

White Portland cements are primarily used in decorative architectural applications, although their properties are similar to that of gray cements. See Table III, where the "Exemplary Prior Cement" is manufactured according to the '547 patent, and is commercially available. The expense to exclude iron oxide from their formula makes them more expensive and more difficult to manufacture.

| Table III | |
|---|---|
| Physical Properties of Cement Sample | |
| White | Exemplary Prior |
| Property | Cement |
| Surface area (m²/kg) | 451 |

| Particle Size distribution | |
|---|---|
| 90% finer than (µm) | 27 |
| 50% finer than (µm) | 9.4 |
| 10% finer than (µm) | 1.85 |
| Setting time, initial (min.) | 47 |
| Setting time, final (min.) | 332 |

| Compressive strength (psi) | |
|---|---|
| after 1 day | 1,550 |
| after 3 days | 3,900 |
| after 7 days | 5,300 |

| Sulfate, weight % of cement: | |
|---|---|
| as gypsum, CaSO₄.2H₂O | 0.2 |
| (K₂SO₄.CaSO₄.H₂O) | |
| % plaster (hemi-hydrate) | 86 |
| *(calculated as % SO3*) | |

According to the invention, fluoride can also be added to a Portland cement in the form of calcium fluoride. Additions of 1.7 wt% fluorine in the cement before firing, increase the strength (at 28 days of setting) about 10%. The fluoride may or may not be released from such a cement.

Some dental applications do not require high radiopacity, such as pulp capping. The sealing and capacity for dentinal bridge formation are more important than radiopacity for use of the material in a thin layer required for pulp capping. For added effectiveness in some dental applications, a cement can be made radiopaque. Bismuth oxide slows the setting and strength development. The bismuth oxide imparts a pale yellow color to the mixture because the bismuth oxide is yellow-colored.

Another radiopacifier can be blended with the cement. For instance, a radiopaque glass used for dental composites can be mixed with portland cement, as an alternative to bismuth oxide. The color of the mixture is white. A glass such as Coming 7724 or 7726 can be used. Such glasses are covered under US Patent # 4,920,082 or 4,775.646, respectively. If a fluoride-releasing glass is used, this mixture would be radiopaque and release fluoride ions. The fluoride release would help prevent internal resorption or cervical decay.

A third radiopaque addition would be barium sulfate instead of bismuth oxide. The barium sulfate is not soluble in water; therefore it would not be a toxic heavy metal compound. This material is white and would also avoid gray coloration of the mixture.

Another addition to cement is Bioglass. Bioglass, a patented formula of glass, is known to be biologically active, and encourage bone growth (see US Patent 4,232,972). Its formula is within this compositional range (all percentages being by weight (v4%)) :

| | |
|---|---|
| Silica | 40-62% |
| Soda | 10-32% |
| Calcia | 10-32% |
| Phosphorous pentoxide | 0-12% |
| Calcium fluoride | 0-18% and |
| Boron oxide | 0-20%. |

The Bioglass can be added as coarse powder, about 170 to 140-mesh size. The large, coarse form of the glass has been found to be more conducive to bone growth than a finer size. Bioglass particles could be used as an "aggregate" in a portland cement and create a concrete. Bioglass (see US Pat. No. 4,775,646) is a white powder, and would not color a mixture with white cement. This would be of most interest for filling bony defects or root-end filling where bone re-growth is important. Hench has patented the mixture of Bioglass and cement. See US Patent #4,775,646 to L. Hench et. al for a fluoride-containing Bioglass. US Patent #4,171,544 to L. Hench et. al for bonding to bone with a high surface area porous, silica-rich surface. This teaches about portland cement for BONDING (not as the restorative for) dental implants, and cement mixed with a biologically active glass.

Other compositions of cement can be considered to create a white cement, as long as they do not include iron oxide. For instance, barium oxide can be partially substituted for calcium oxide. This is a new ingredient, not specified in the first Torabinejad patent (#5,415,547). This would create a cement that is inherently radiopaque and needs no further additions.

The fineness of the cement also affects its usefulness in dentistry. For instance the Torabinejad patent refers to cements of Type 3, a relatively fine cement having a surface area of 450 to 550 m²/kg. However, such cements are perceived as grainy or sandy by dentists, having lesser quality, and less packable into fine orifices. The surface area measurement gives a general indication of the fineness of the powder, but does not adequately characterize the distribution of the powder particles sizes.

Two approaches can be followed to improve the performance of such dental materials: removal of coarse particles, or reduction in the average particle size. The removal of coarse particles can be achieved by sieving or air elutriation. The particle size reduction can be achieved by milling processes such as ball milling, air attrition, or attrition milling.

Finer cements are more suitable for either a root canal sealing material or a root canal obturation material. The use of a cement with a surface areas of about 1,000 m²/kg allows it to be easily filled in a root canal, including lateral canals. We tested such a cement for a root canal sealer and found it preferable to a cement have a lower surface area. Furthermore, the removal of particles coarser than 400 mesh (44 µm) improved the handling of the gray cement used in the commercially available ProRoot MTA material. This process increased the measured surface area from 454 to 509 m²/kg.

Therefore, it is apparent that a dental material according to the invention as described above, is useful in meeting the stated objectives of the invention. It will be understood that amount of various components, can be varied and still fall within the scope of the invention. The scope of the invention will be determined only by the claims.

## Claims

1. A dental material selected from a cement and a restorative material, which comprises Portland cement and which contains
(a) from 61 to 70 wt.-% calcia,
(b) from 19 to 29 wt.-% silica,
(c) from 5 to 15 wt.-% alumina and
(d) less than 0.5 wt.-% iron oxide,
based upon 100 wt.-% of the material.

2. The dental material as in claim 1 which is white in color.

## Patentansprüche

1. Dentalmaterial, ausgewählt aus einem Zement und einem Verstärkungsmaterial, umfassend Portlandzement und enthaltend
(a) 61 bis 70 Gew.-% Calcia,
(b) 19 bis 29 Gew.-% Siliciumdioxid,
(c) 5 bis 15 Gew.-% Aluminiumoxid,
(d) weniger als 0,5 Gew.-% Eisenoxid,
bezogen auf 100 Gew.-% des Materials.

2. Dentalmaterial nach Anspruch 1, das eine weiße Farbe aufweist.

## Revendications

1. Matériau dentaire choisi parmi un ciment et un matériau pour restauration, comprenant du ciment Portland et contenant
(a) de 61 à 70% en poids de Calcia,
(b) de 19 à 29% en poids de silice,
(c) de 5 à 15% en poids d'alumine et
(d) moins de 0,5% en poids d'oxyde de fer,
sur la base de 100% en poids du matériau.

2. Matériau dentaire selon la revendication 1, qui est de couleur blanche.
